(19) Europäisches Patentamt
European Patent Office
Office européen des brevets

(11) EP 0 583 591 B1

(12) EUROPÄISCHE PATENTSCHRIFT

(45) Veröffentlichungstag und Bekanntmachung des Hinweises auf die Patenterteilung:
**09.10.1996 Patentblatt 1996/41**

(51) Int Cl.[6]: **C07D 303/14**, C09K 19/58, C07B 57/00

(21) Anmeldenummer: **93110709.8**

(22) Anmeldetag: **05.07.1993**

# (54) Verfahren zur Herstellung hoch enantiomerenreiner Oxiranalkohole

Process for the preparation of oxiranalcohols of high enantiomeric purity

Procédé pour la préparation d'alcools oxiraniques a haute pureté énantiomérique

(84) Benannte Vertragsstaaten:
**CH DE FR GB LI**

(30) Priorität: **30.07.1992 DE 4225155**

(43) Veröffentlichungstag der Anmeldung:
**23.02.1994 Patentblatt 1994/08**

(73) Patentinhaber: **HOECHST AKTIENGESELLSCHAFT**
**65926 Frankfurt am Main (DE)**

(72) Erfinder: **Manero, Javier, Dr.**
**D-6230 Frankfurt/Main (DE)**

(56) Entgegenhaltungen:

**EP-A- 0 454 463**
**EP-A- 0 461 285**
**EP-A- 0 515 142**
**GB-A- 2 247 020**
**US-A- 4 638 073**
**US-A- 5 178 793**

- **JOURNAL OF THE AMERICAN CHEMICAL SOCIETY Bd. 109, Nr. 19 , 6. September 1987 , WASHINGTON DC (US) Seiten 5765 - 5780 Y. GAO ET AL. 'Catalytic asymmetric epoxidation and kinetic resolution: Modified procedures including in situ derivatization'**
- **TETRAHEDRON LETTERS Bd. 28, Nr. 24 , 1987 , OXFORD (GB) Seiten 2709 - 2712 S. TSUBOI ET AL. 'A practical synthesis of chiral 3-chloro-2-hydroxyalkanoates and 2,3-epoxyalcohols'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 583 591 B1

Printed by Jouve, 75001 PARIS (FR)

## Beschreibung

Schon seit beinahe 150 Jahren sind Chemiker bemüht, chirale Verbindungen in weitgehend enantiomerenreiner Form darzustellen, sei es durch Synthese eines einzigen oder durch Trennung der beiden Enantiomeren. Eine Verbindung ist chiral, wenn sie mit ihrem Spiegelbild nicht zur Deckung gebracht werden kann. Bild und Spiegelbild nennt man die beiden Enantiomere der Verbindung oder auch optische Antipoden. Chirale Verbindungen sind im allgemeinen optisch aktiv, d. h. sie drehen die Ebene von polarisiertem Licht um einen bestimmten Betrag. Dieser Betrag ist bei beiden Enantiomeren gleich groß, besitzt aber unterschiedliche Vorzeichen.

Besonders im Bereich der Biochemie ist es bekannt, daß von den beiden Enantiomeren nur eines eine bestimmte Wirkung zeigt, bzw. daß beide sogar vollkommen unterschiedliche Wirkungen haben können. Am bekanntesten ist sicherlich das Beispiel des Schlafmittels Thalidomid, bei dem das eine Enantiomer als Schlafmittel wirkt, das andere aber teratogen ist.

Aber auch in vielen anderen Bereichen von Chemie und Technik, wie Pflanzenschutz und NLO, ist es wichtig, mit möglichst enantiomerenreinen Verbindungen zu arbeiten. Ein weiteres Beispiel dafür sind ferroelektrische Flüssigkristalle, d. h. solche, die chirale Moleküle enthalten und geneigte smektische Phasen ausbilden.

Clark und Lagerwall konnten zeigen, daß die Verwendung ferroelektrischer Flüssigkristallsysteme in sehr dünnen Zellen zu optoelektrischen Schalt- oder Anzeigeelementen führt, die im Vergleich zu den herkömmlichen TN ("twisted nematic")-Zellen um bis zu einem Faktor 1000 schnellere Schaltzeiten haben (vgl. z. B. Lagerwall et al. "Ferroelectric Liquid Crystals for Displays", SID Symposium, October Meeting 1985, San Diego, Ca., (USA). Aufgrund dieser und anderer günstiger Eigenschaften, z. B. der bistabilen Schaltmöglichkeit und des nahezu blickwinkelunabhängigen Kontrasts, sind FLCs grundsätzlich für die obengenannten Anwendungsgebiete, z. B. über eine Matrixansteuerung, gut geeignet.

Die optische Schaltzeit $\tau$ [µs] ferroelektrischer Flüssigkristallsysteme, die möglichst kurz sein soll, hängt von der Rotationsviskosität des Systems $\gamma$ [mPas], der spontanen Polarisation $P_s$[nC/cm$^2$] und der elektrischen Feldstärke E [V/m] ab nach der Beziehung:

$$\tau \sim \frac{\gamma}{P_S \cdot E}$$

Der mit einem gegebenen chiralen Material erreichte $P_s$-Wert korreliert mit der optischen Reinheit des Materials.

$$P_s \sim \text{optische Reinheit}$$

Die optische Reinheit (enantiomeric excess, ee) ist definiert als

$$ee = \frac{A_1 - A_2}{A_1 + A_2} \cdot 100$$

wobei $A_1$ die Menge des Enantiomeren ist, welches im Überschuß vorhanden ist, und $A_2$ die Menge des optischen Antipoden. Es ist daher erwünscht, ausschließlich ein einziges Enantiomer einer Substanz in ferroelektrischen Mischungen einzusetzen, um den maximal möglichen $P_s$-Wert mit den eingesetzten Substanzen zu erreichen.

Substanzen, die sich als Dotierstoffe in ferroelektrischen Flüssigkristallmischungen einsetzen lassen sind beispielsweise Oxiranether (EP 263 437) und/oder Oxiranester (EP 292 954). Die Oxiranester können z. B. aus Oxiranalkoholen über die zugehörigen Oxirancarbonsäuren erhalten werden (z. B. Carlsen et al., J. Org. Chem., 46 (1981) 3936), auch können Oxiranalkohole mit Carbonsäurederivaten zu entsprechenden Estern umgesetzt werden (US-A 4,638,073).

Zur Darstellung von Oxiranalkoholen in weitgehend enantiomerenreiner Form sind zahlreiche Verfahren bekannt. Die enantioselektive Synthese von Oxiranalkoholen aus Olefinen ist beispielsweise in EP-0 046 033 oder in J. Am. Chem. Soc., 109 (1987) 5765 beschrieben. Trotz der guten Enantio- und Diastereoselektivität der asymmetrischen Epoxidierung werden aber keine ee-Werte größer als 98 % (nach NMR) erreicht. In der EP-A 454 463 ist ein Verfahren zur Herstellung von Epoxyalkoholen mit hoher Enantiomerenreinheit beschrieben, bei dem ein Enantionmer durch stereoselektive Umesterung in Gegenwart einer Lipase abgetrennt wird.

Die weitere "optische Reinigung" von Oxiranalkoholen kann grundsätzlich durch Umsetzung mit einem chiralen Hilfsstoff erfolgen, wobei aus dem Enantiomeren- ein Diastereomerenpaar wird, das durch physikalische Methoden, wie Destillation, Chromatographie oder Umkristallisation, getrennt werden kann. Durch die Notwendigkeit der Bildung

und anschließenden Zersetzung von Derivaten sind diese Verfahren jedoch grundsätzlich viel aufwendiger als die mit der reinen Substanz arbeitenden.

Über Versuche zur direkten Trennung der Enantiomeren von (2S,3S)-2,3-Epoxy-1-hexanol durch Destillation, Chromatographie oder durch gewöhnliches Umkristallisieren wurde berichtet (siehe Gao et al., J. Org. Chem., 53 (1988) 4114), jedoch wurden nach diesen Methoden keine ee-Werte von größer als 98 % ee erzielt.

Weiterhin ist es bekannt, enantiomerenreine Verbindungen durch Kristallisation zu erhalten, indem der Lösung beide Enantiomere Impfkristalle des gewünschten Enantiomeren zugesetzt werden (siehe z. B. A. Collet et al., Bull. Soc. Chim. 1972, 127). Diese sogenannte spontane Racematspaltung gelingt jedoch nur, wenn die racemische Mischung als Konglomerat, d. h. als ein Gemisch von Kristallen aus jeweils einem Enantiomeren, kristallisiert, was nur wenige Verbindungen tun.

Es bestand also die Aufgabe, ein Verfahren bereitzustellen, mit dem Oxiranalkohole einer noch höheren Enantiomerenreinheit erhalten werden.

Überraschenderweise wurde nun gefunden, daß man Oxiranalkohole ohne nachweisbare Verunreinigung durch die anderen Diastereomere oder Enantiomere durch Umkristallisation in geeigneten Lösungsmitteln bei Temperaturen von 0,05 bis 10°C unterhalb des Schmelzpunktes des Oxiranalkohols erhalten kann. Das Intervall zwischen Schmelzpunkt und der Temperatur der Umkristallisation kann umso breiter sein, je höher der Schmelzpunkt der Verbindung liegt.

Gegenstand der Erfindung ist daher ein Verfahren zur Herstellung hochenantiomerenreiner Oxiranalkohole, dadurch gekennzeichnet, daß eine Mischung isomerer Oxiranalkohole, in der das gewünschte Enantiomere in einem Überschuß von mindestens 60 % ee vorliegt, in einem Lösungsmittel oder Lösungsmittelgemisch bei einer Temperatur von 0,05 bis 10°C unterhalb des Schmelzpunkts des Oxiranalkohols umkristallsiert wird.

Weiterhin Gegenstand der Erfindung ist ein Verfahren zur Herstellung von Dotierstoffen für ferroelektrische Flüssigkristallmischungen, dadurch gekennzeichnet, daß eine Mischung von isomeren Oxiranalkoholen der Formel (I), in der daß gewünschte Enantiomere in einem Überschuß von mindestens 60% ee vorliegt, in einem Lösungsmittel oder Lösungsmittelgemisch bei einer Temperatur von 0,05 bis 10°C unterhalb des Schmelzpunktes des Oxiranalkohols umkristallisiert wird und anschließend, gegebenenfalls nach Umsetzung zur entsprechenden Oxirancarbonsäure, durch Veretherung bzw. Veresterung zu einem Dotierstoff für ferroelektrische Flüssigkristalle umgesetzt wird.

Erfindungsgemäß dargestellte Oxiranalkohole zeichnen sich durch eine hohe Enantiomerenreinheit aus. Ferroelektrische Flüssigkristalle, die erfindungsgemäß hochenantiomerenreine Oxiranalkohole und/oder von diesen abgeleitete Derivate enthalten, haben besonders kurze Schaltzeiten.

Als Oxiranalkohole werden vorzugsweise Verbindungen der Formel I eingesetzt

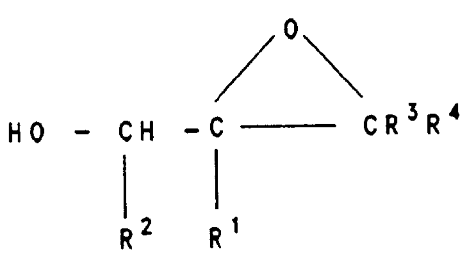

I

- $R^1$ ist Wasserstoff oder ein geradkettiger oder verzweigter Alkylrest mit 1 bis 18 C-Atomen (mit oder ohne asymmetrisches C-Atom), wobei auch eine oder mehrere $-CH_2$-Gruppen durch -O-, -CO-, -CH=CH-, -C≡C-, Δ, $-Si(CH_3)_2$- oder 1,4-Cyclohexylen ersetzt sein können, mit der Maßgabe, daß Sauerstoffatome nicht unmittelbar aneinander gebunden sein dürfen, und der Maßgabe, daß mindestens $R^3$ oder $R^4$ von Wasserstoff verschieden sein muß, wenn $R^1$ ungleich Methyl ist, und der Maßgabe, daß weder -O-, -CO-, -CH=CH- oder -C≡C- direkt an den Oxiranring gebunden sein dürfen, und/oder ein oder mehrere H-Atome des Alkylrestes durch -F, -Cl, -Br, - SCN, -OCN oder $-N_3$ substituiert sein können.

- $R^2$ ist Wasserstoff oder ein geradkettiger oder verzweigter Alkylrest mit 1 bis 14 C-Atomen (mit oder ohne asymmetrisches C-Atom), wobei auch eine oder mehrere $-CH_2$-Gruppen durch -O-, Δ, $-Si(CH_3)_2$- oder 1,4-Cyclohexylen ersetzt sein können, mit der Maßgabe, daß Sauerstoffatome nicht unmittelbar aneinander gebunden sein dürfen, und/oder ein oder mehrere H-Atome des Alkylrestes durch -F, -Cl, -Br, -SCN, -OCN oder $-N_3$ substituiert sein können.

- $R^3$, $R^4$ sind Wasserstoff oder ein geradkettiger oder verzweigter Alkylrest mit 1 bis 18 C-Atomen (mit oder ohne asymmetrisches C-Atom), wobei auch eine oder mehrere $-CH_2$-Gruppen durch -O-, -CO-, -CH=CH-, -C≡C-,

Δ, - $Si(CH_3)_2$- oder 1,4-Cyclohexylen ersetzt sein können, mit der Maßgabe, daß Sauerstoffatome nicht unmittelbar aneinander gebunden sein dürfen, und der Maßgabe, daß weder -O-, -CO-, -CH=CH- oder -C≡C- direkt an den Oxiranring gebunden sein dürfen, und/oder ein oder mehrere H-Atome des Alkylrestes durch -F, -Cl, -Br, -SCN, -OCN oder -$N_3$ substituiert sein können.

$R^1$ und $R^3$, $R^1$ und $R^4$ sowie $R^3$ und $R^4$ können zusammen auch einen Ring bilden.

Besonders bevorzugt ist

$R^1$ ist Wasserstoff oder ein geradkettiger oder verzweigter Alkylrest mit 1 bis 14 C-Atomen (mit oder ohne asymmetrisches C-Atom), wobei auch eine oder mehrere -$CH_2$-Gruppen durch -O-, -CH=CH- Δ, oder -$Si(CH_3)_2$- ersetzt sein können, mit der Maßgabe, daß Sauerstoffatome nicht unmittelbar aneinander gebunden sein dürfen, und der Maßgabe, daß mindestens $R^3$ oder $R^4$ von Wasserstoff verschieden sein muß, wenn $R^1$ ungleich Methyl ist, und der Maßgabe, daß weder -O-, noch -CH=CH- direkt an den Oxiranring gebunden sein dürfen, und/oder ein oder mehrere H-Atome des Alkylrestes durch -F, -Cl oder -Br substituiert sein können,

$R^2$ Wasserstoff oder ein geradkettiger oder verzweigter Alkylrest mit 1 bis 10 C-Atomen (mit oder ohne asymmetrisches C-Atom), wobei auch eine oder mehrere -$CH_2$-Gruppen durch -O- oder Δ ersetzt sein können, mit der Maßgabe, daß Sauerstoffatome nicht unmittelbar aneinander gebunden sein dürfen, und/oder ein oder mehrere H-Atome des Alkylrestes durch -F oder -Cl substituiert sein können,

$R^3$, $R^4$ Wasserstoff oder ein geradkettiger oder verzweigter Alkylrest mit 1 bis 14 C-Atomen (mit oder ohne asymmetrisches C-Atom), wobei auch eine oder mehrere -$CH_2$-Gruppen durch -O-, -CH=CH- Δ oder -$Si(CH_3)_2$- ersetzt sein können, mit der Maßgabe, daß Sauerstoffatome nicht unmittelbar aneinander gebunden sein dürfen, und der Maßgabe, daß weder -O- noch -CH=CH- direkt an den Oxiranring gebunden sein dürfen, und/oder ein oder mehrere H-Atome des Alkylrestes durch -F, -Cl, oder -Br substituiert sein können.

$R^1$ und $R^3$, $R^1$ und $R^4$ sowie $R^3$ und $R^4$ können zusammen auch einen Ring bilden.

Ganz besonders bevorzugt ist

$R^1$ Wasserstoff oder ein geradkettiger oder verzweigter Alkylrest mit 1 bis 12 C-Atomen (mit oder ohne asymmetrisches C-Atom), wobei auch eine oder mehrere -$CH_2$-Gruppen durch -O- ersetzt sein können, mit der Maßgabe, daß Sauerstoffatome nicht unmittelbar aneinander gebunden sein dürfen, und der Maßgabe, daß mindestens $R^3$ oder $R^4$ von Wasserstoff verschieden sein muß, wenn $R^1$ ungleich Methyl ist, und der Maßgabe, daß -O- nicht direkt an den Oxiranring gebunden sein darf, und/oder ein oder mehrere H-Atome des Alkylrestes durch -F substituiert sein können,

$R^2$ Wasserstoff oder ein geradkettiger oder verzweigter Alkylrest mit 1 bis 6 C-Atomen (mit oder ohne asymmetrisches C-Atom), wobei ein oder mehrere H-Atome des Alkylrestes durch -F substituiert sein können.

$R^3$, $R^4$ Wasserstoff oder ein geradkettiger oder verzweigter Alkylrest mit 1 bis 12 C-Atomen (mit oder ohne asymmetrisches C-Atom), wobei auch eine oder mehrere -$CH_2$-Gruppen durch -O-, Δ oder -$Si((CH_3)_2$- ersetzt sein können, mit der Maßgabe, daß Sauerstoffatome nicht unmittelbar aneinander gebunden sein dürfen, und der Maßgabe, daß -O- nicht direkt an den Oxiranring gebunden sein darf, und/oder ein oder mehrere H-Atome des Alkylrestes durch -F substituiert sein können.

$R^1$ und $R^3$, $R^1$ und $R^4$ sowie $R^3$ und $R^4$ können zusammen auch einen Ring bilden.

Insbesondere sind $R^1$, $R^2$ und $R^3$ Wasserstoff und $R^4$ Alkyl, vor allem bevorzugt ist $R^4$ Ethyl, Propyl, Butyl oder Pentyl.

Es können prinzipiell die (S,S), (R,R), (S,R) und (R,S) Isomeren der obengenannten Verbindungen erhalten werden.

Die Oxiranalkohole der Formel (I) werden nach an sich bekannten Literaturmethoden dargestellt; solche Synthesen sind beispielsweise in EP 046 033 oder in J. Am. Chem. Soc. 109 (1987) 5765 beschrieben.

Mit dem erfindungsgemäßen Verfahren können die oben angegebenen Oxiranalkohole in optischen Reinheiten von > 99,5 % ee dargestellt werden. Um in dem erfindungsgemäßen Verfahren Anwendung finden zu können, müssen die Oxiranalkohole der Formel (I) das gewünschte Enantiomer im Überschuß von mindestens 60 % ee enthalten, vorzugsweise sollte das gewünschte Enantiomer in einem Überschuß von 70 % ee, insbesondere von 80 % ee, vorliegen.

Der zu reinigende Oxiranalkohol wird in einem geeigneten organischen Lösungsmittel, vorzugsweise bei erhöhten Temperaturen, insbesondere bei der Siedetemperatur des Lösungsmittels, gelöst. Als Lösungsmittel finden vorzugsweise aliphatische und aromatische Kohlenwasserstoffe, wie Pentan, Hexan, Heptan, Cyclohexan, Methylcyclohexan, Benzol, Toluol und Xylole, halogenierte aliphatische und aromatische Kohlenwasserstoffe, wie Dichlormethan, Chloroform, Tetrachlorkohlenstoff, 1,2-Dichlorethan und Chlorbenzol, cyclische und acyclische Ether, wie Diethylether, Dibutylether, Tetrahydrofuran und 1,4-Dioxan, sowie Ester, wie Ethylacetat, oder Amide, wie Dimethylformamid, Anwendung. Besonders bevorzugt ist die Verwendung von Alkanen, insbesondere n-Alkanen. Es können auch Mischungen der genannten Lösungsmittel verwendet werden. Die Konzentration an Oxiranalkohol in der Lösung liegt zwischen 0,1 Gramm pro Liter Lösung bis zur Sättigungskonzentration beim Siedepunkt des Lösungsmittels. Bevorzugt sind Konzentrationen zwischen 1 Gramm Oxiranalkohol pro Liter Lösungsmittel und der Sättigungskonzentration des Oxiranalkohols beim Siedepunkt des Lösungsmittels, besonders bevorzugt zwischen 20 Gramm Oxiranalkohol pro Liter Lösungsmittel und der Sättigungskonzentration des Oxiranalkohols beim Siedepunkt des Lösungsmittels. Ganz besonders bevorzugt sind Konzentrationen zwischen 50 Gramm Oxiranalkohol pro Liter Lösungsmittel und 500 Gramm Oxiranalkohol pro Liter Lösungsmittel, bzw. der Sättigungskonzentration beim Siedepunkt des Lösungsmittels, falls diese geringer ist.

Nach dem Auflösen des Oxiranalkohols erfolgt gegebenenfalls eine Filtration, um ungelöste Verunreinigungen abzutrennen.

Anschließend wird die Lösung für einen bestimmten Zeitraum, vorzugsweise 48 Stunden, insbesondere 18 Stunden, bei einer Temperatur von 0,05 bis 10°C unterhalb des Schmelzpunktes des Oxiranalkohols gehalten. Bevorzugt sind Temperaturen 0,1 bis 5,0 Grad unterhalb des Schmelzpunktes.

Das gewünschte Enantiomer kristallisiert nach einiger Zeit in reiner Form aus. Es wird beispielsweise durch Absaugen, gegebenenfalls unter Kühlung, isoliert.

Die Bestimmung der optischen Reinheit, d. h. des ee-Wertes, kann nach verschiedenen, dem Fachmann geläufigen Methoden erfolgen. Grundsätzlich kann ein Enantiomerengemisch mit chiralen oder achiralen Methoden untersucht werden. Bei den chiralen werden diastereomere Wechselwirkungen beobachtet, bei den achiralen wird das Enantiomerengemisch durch Reaktion mit einem chiralen Hilfsstoff zu diastereomeren Verbindungen umgesetzt, die sich aufgrund ihrer unterschiedlichen physikalischen und chemischen Eigenschaften analysieren lassen.

Als chirale Hilfsstoffe eignen sich je nach Funktionalisierung des Enantiomerengemisches verschiedene enantiomerenreine Naturstoffe (Alkohole, Amine, Aminosäuren, Säuren, etc.) und speziell entwickelte Verbindungen, z. B. Moshers Säure: $\alpha$-Methoxy-$\alpha$-trifluormethyl-a-phenylessigsäure. Die quantitative Analyse der Diastereomeren kann beispielsweise mit NMR-Spektroskopie, GC oder HPLC durchgeführt werden.

Als chirale Methoden kommen z. B. in Frage

a) die Drehwertmessung, wobei allerdings der wellenlängenabhängige Drehwert des reinen Enantiomers bekannt und deutlich von Null verschieden sein und das Enantiomerengemisch in ausreichenden Mengen analysenrein zur Verfügung stehen muß;

b) die NMR-spektroskopische Vermessung des Enantiomerengemischs in einem chiralen bzw. chiral dotierten Medium, wie sie z. B. mit chiralen Europiumkomplexen, wie $Eu(tfc)_3$, $Eu(hfc)_3$, erfolgen kann, oder

c) die Gaschromatographie an chiralen Säulen, z. B. an Cyclodertrinen, wie u. a. bei V. Schurig, D. Wistuba, Angew. Chem. 98 (1986) 1008 und W. A. König et. al, Angew. Chem. 101 (1989) 180 beschrieben. Diese Methode ist aufgrund ihrer sehr hohen Präzision und Empfindlichkeit (ee-Werte größer 99 % können auf vier gültigen Stellen bestimmt werden) prinzipiell sehr gut geeignet. Bei dieser Methode kann es vorteilhaft sein, das zu untersuchende Enantiomerengemisch in geeignete flüchtige bzw. chemisch oder physikalisch stabile Derivate, wie Silyl-, Acetyl- oder Trifluormethyl-Verbindungen, zu überführen.

Die erfindungsgemäß dargestellten hochenantiomerenreinen Oxiranalkohole können in allen Bereichen eingesetzt werden, in denen Verbindungen dieses Typs von Interesse sind, wie in der pharmazeutischen Chemie, in der Chemie der Pflanzenschutzmittel oder im Bereich der nichtlinearen Optik. Bevorzugt ist eine Verwendung als Ausgangsmaterial für Substanzen, die sich als Dotierstoffe für ferroelektrische Flüssigkristallmischungen eignen, z. B. Oxiranether und Oxiranester.

Die Erfindung wird durch die Beispiele näher erläutert:

Beispiel 1:

Synthese von (2S,3R)-Propyloxiranmethanol

19,56 g gepulvertes Molsieb (3 Å), 1,5 l Dichlormethan, 17,9 ml (60 mmol) Titanisopropylat und 12,36 ml (36,01 mmol) L (+)-Diethyltartrat werden bei 0 °C unter $N_2$ in einem 4 l 4-Halskolben vorgelegt. Zu der gerührten Suspension

werden bei - 20 °C 200 ml (1 mol) t-Butylhydroperoxid (5 M in Heptan) innerhalb von 20 min zugetropft. Man rührt 20 min bei - 20 °C und tropft dann eine Lösung von 50 g (500 mmol) cis-2-Hexen-1-ol in 50 ml Dichlormethan zu. Anschließend wird 20 Stunden bei - 5 bis - 10 °C gehalten. Man vesetzt bei 0 °C mit einer Lösung von 165,3 g (60,13 mmol) $FeSO_4 \bullet 7H_2O$ und 50,08 g (300,4 mmol) L (+)-Weinsäure in 770 ml $H_2O$, trennt die Phasen und extrahiert die wäßrige Phase 3 x mit je 250 ml Diethylether. Die vereinigten organischen Phasen werden über $MgSO_4$ getrocknet, über eine Glasfritte abgesaugt und am Rotationsverdampfer eingeengt. Man nimmt in 1,3 l Diethylether auf, versetzt unter schnellem Rühren bei 0 - 4 °C innerhalb von 15 min mit einer Lösung von 24,6 g NaOH in 350 ml gesättigter wäßriger NaCl-Lösung und rührt 1 Stunde bei 0 bis 3 °C nach. Man versetzt mit 500 ml $H_2O$, trennt die Phasen und extrahiert die wäßrige Phase 5 x mit je 250 ml Diethylether. Die vereinigten organischen Phasen werden über $MgSO_4$ getrocknet, über eine Glasfritte abgesaugt und das Lösungsmittel am Rotationsverdampfer abgezogen. Das so erhaltene Öl wird 45 min an der Ölpumpe getrocknet.
Rohausbeute: 56,13 g. Nach Säulenchromatographie (Kieselgel, Heptan/Ethylacetat : 2/1) erhält man 46,7 g (2S,3R)-3-Propyloxiranmethanol einer optischen Reinheit von 81,1 % ee (GC). Schmelzpunkt: - 6 °C.

Beispiel 2:

10 Gramm (2S,3R)-3-Propyloxiranmethanol aus Beispiel 1 werden in 100 ml Hexan gelöst und 12 Stunden bei - 8 °C gehalten. Es fallen weiße Kristalle aus, die bei der genannten Temperatur abgesaugt werden.

Beispiel 3:

100 mg (2S,3R)-3-Propyloxiranmethanol aus Beispiel 2 werden mit 400 mg N-Methyl-bis-(trifluoracetyl)-amid (MBTFA) sowie 1 ml Dichlormethan in ein druckstabiles 2 ml Fläschchen gefüllt und verkapselt. Nach 30 min Erhitzen bei 100 °C wird 1 µl der Derivatlösung in einen Gaschromatographen (Dani 8500, Firma Dani) injiziert.

| | | |
|---|---|---|
| Säule: | 50 m Fused Silica Kapillarsäule mit 0,25 mm Innendurchmesser. | |
| Stationäre Phase: | 0,25 µm Permethyliertes β-Cydodextrin (RLipodex β-PM, Firma Macherey + Nagel) | |
| GC-Bedingungen: | Trägergas: | Helium mit 1,5 bar Vordruck = 3 ml/min |
| | Injektor: | Split-Injection bei 250 °C |
| | Detektor: | 100 ml/min |
| | Ofen: | 100 °C |

Im Gegensatz zu Materialien aus Beispiel 1 und dem Vergleichsbeispiel wird mit dem erfindungsgemäß umkristallisierten Material aus Beispiel 2 nur ein einziges Signal erhalten. Mit dem erfindungsgemäßen Verfahren werden also enantiomerenreine Verbindungen erhalten.

Vergleichsbeispiel:

10 g (2S,3R)-3-Propyloxiranmethanol, erhalten nach Beispiel 1, werden in 100 ml Hexan gelöst und bei - 30 °C umkristallisiert. Das erhaltene Produkt ist bezüglich der chiralen Reinheit identisch mit dem eingesetzten Ausgangsmaterial.

## Patentansprüche

1. Verfahren zur Herstellung hochenantiomerenreiner Oxiranalkohole, dadurch gekennzeichnet, daß eine Mischung der isomeren Oxiranalkohole, in der das gewünschte Enantiomere in einem Überschuß von mindestens 60 % ee vorliegt, in einem Lösungsmittel oder Lösungsmittelgemisch bei einer Temperatur von 0,05 bis 10°C unterhalb des Schmelzpunkts des Oxiranalkohols umkristallisiert wird.

2. Verfahren nach Anspruch 1, dadurch gekennzeichnet, daß als Oxiranalkohol eine Verbindung der Formel (I) eingesetzt wird, wobei

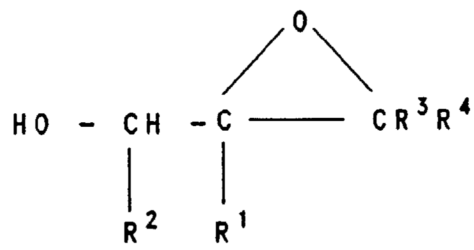

I

$R^1$ Wasserstoff ist oder ein geradkettiger oder verzweigter Alkylrest mit 1 bis 18 C-Atomen (mit oder ohne asymmetrisches C-Atom), wobei auch eine oder mehrere $-CH_2$-Gruppen durch -O-, -CO-, -CH=CH-, -C≡C-, Δ, $-Si(CH_3)_2$- oder 1,4-Cyclohexylen ersetzt sein können, mit der Maßgabe, daß Sauerstoffatome nicht unmittelbar aneinander gebunden sein dürfen, und der Maßgabe, daß mindestens $R^3$ oder $R^4$ von Wasserstoff verschieden sein muß, wenn $R^1$ ungleich Methyl ist, und der Maßgabe, daß weder -O-, -CO-, -CH =CH- oder -C≡C- direkt an den Oxiranring gebunden sein dürfen, und/oder ein oder mehrere H-Atome des Alkylrestes durch -F, -Cl, -Br, -SCN, -OCN oder $-N_3$ substituiert sein können;

$R^2$ Wasserstoff ist oder ein geradkettiger oder verzweigter Alkylrest mit 1 bis 14 C-Atomen (mit oder ohne asymmetrisches C-Atom), wobei auch eine oder mehrere $-CH_2$-Gruppen durch -O-, Δ, $-Si(CH_3)_2$- oder 1,4-Cyclohexylen ersetzt sein können, mit der Maßgabe, daß Sauerstoffatome nicht unmittelbar aneinander gebunden sein dürfen, und/oder ein oder mehrere H-Atome des Alkylrestes durch -F, -Cl, -SCN, -OCN oder $-N_3$ substituiert sein können;

$R^3$, $R^4$ Wasserstoff sind oder ein geradkettiger oder verzweigter Alkylrest mit 1 bis 18 C-Atomen (mit oder ohne asymmetrisches C-Atom), wobei auch eine oder mehrere $-CH_2$-Gruppen durch -O-, -CO-, Δ, -CH=CH-, -C≡C-, $-Si(CH_3)_2$- oder 1,4-Cyclohexylen ersetzt sein können, mit der Maßgabe, daß Sauerstoffatome nicht unmittelbar aneinander gebunden sein dürfen, und der Maßgabe, daß weder -O-, -CO-, -CH=CH- oder -C≡C- direkt an den Oxiranring gebunden sein dürfen, und/oder ein oder mehrere H-Atome des Alkylrestes durch -F, -Cl, -Br, -SCN, -OCN oder $-N_3$ substituiert sein können, und

$R^1$ und $R^3$, $R^1$ und $R^4$ sowie $R^3$ und $R^4$ zusammen auch einen Ring bilden können.

**3.** Verfahren nach Anspruch 1 oder 2, dadurch gekennzeichnet, daß mindestens ein Lösungsmittel aus der Gruppe aliphatische und aromatische Kohlenwasserstoffe, halogenierte aliphatische und aromatische Kohlenwasserstoffe, Ether, Ester und Amide verwendet wird.

**4.** Verfahren zur Herstellung von Dotierstoffen für ferroelektrische Flüssigkristallmischungen, dadurch gekennzeichnet, daß eine Mischung von isomeren Oxiranalkoholen der Formel (I) in Anspruch 2, in der das gewünschte Enantiomere in einem Überschuß von mindestens 60% ee vorliegt, in einem Lösungsmittel oder Lösungsmittelgemisch bei einer Temperatur von 0,05 bis 10°C unterhalb des Schmelzpunktes des Oxiranalkohols umkristallisiert wird und anschließend, gegebenenfalls nach Umsetzung zur entsprechenden Oxirancarbonsäure, durch Veretherung bzw. Veresterung zu einem Dotierstoff für ferroelektrische Flüssigkristalle umgesetzt wird.

## Claims

**1.** A process for preparing highly pure enantiomers of oxirane alcohols, which comprises recrystallizing a mixture of the isomeric oxirane alcohols, in which the desired enantiomer is present in an excess of at least 60% ee, in a solvent or solvent mixture at a temperature from 0.05 to 10°C below the melting point of the oxirane alcohol.

**2.** The process as claimed in claim 1, wherein the oxirane alcohol used is a compound of formula (I),

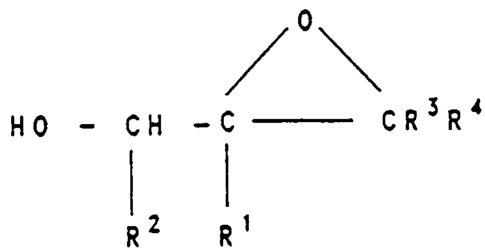

I

where

R1 is hydrogen or a straight-chain or branched alkyl radical having 1 to 18 carbon atoms (with or without an asymmetric carbon atom), it also being possible for one or more $-CH_2-$ groups to be replaced by -O-, -CO-, -CH=CH-, -C≡C-, Δ, $-Si(CH_3)_2-$ or 1,4-cyclohexylene, with the proviso that oxygen atoms must not be bound directly to one another, and with the proviso that at least $R^3$ or $R^4$ must be other than hydrogen if $R^1$ is not methyl and with the proviso that neither -O-, -CO-, -CH=CH- nor -C≡C- must be bound directly to the oxirane ring, and/or one or more hydrogen atoms of the alkyl radical can be substituted by -F, -Cl, -Br, -SCN, -OCN or $-N_3$;

R2 is hydrogen or a straight-chain or branched alkyl radical having 1 to 14 carbon atoms (with or without an asymmetric carbon atom), it also being possible for one or more $-CH_2-$ groups to be replaced by -O-, Δ, -Si $(CH_3)_2-$ or 1,4-cyclohexylene, with the proviso that oxygen atoms must not be bound directly to one another, and/or one or more hydrogen atoms of the alkyl radical can be substituted by -F, -Cl, -SCN, -OCN or $-N_3$;

$R^3$ and $R^4$ are hydrogen or a straight-chain or branched alkyl radical having 1 to 18 carbon atoms (with or without an asymmetric carbon atom), it also being possible for one or more $-CH_2-$ groups to be replaced by -O-, -CO-, Δ, -CH=CH-, -C≡C-, $-Si(CH_3)_2-$ or 1,4-cyclohexylene, with the proviso that oxygen atoms must not be bound directly to one another, and with the proviso that neither -O-, -CO-, -CH=CH- nor -C≡C- must be bound directly to the oxirane ring, and/or one or more hydrogen atoms of the alkyl radical can be substituted by -F, -Cl, -Br, -SCN, -OCN or $-N_3$, and

$R^1$ and $R^3$, $R^1$ and $R^4$ as well as $R^3$ and $R^4$ together can also form a ring.

**3.** The process as claimed in claim 1 or 2, wherein at least one solvent from the group of aliphatic and aromatic hydrocarbons, halogenated aliphatic and aromatic hydrocarbons, ethers, esters and amides is used.

**4.** A process for preparing doping materials for ferroelectric liquid crystal mixtures, which comprises recrystallizing a mixture of isomeric oxirane alcohols of formula (I) in claim 2, in which the desired enantiomer is present in an excess of at least 60% ee, in a solvent or solvent mixture at a temperature from 0.05 to 10°C below the melting point of the oxirane alcohol and subsequently reacting the mixture by etherification or esterification to give a doping material for ferroelectric liquid crystals, if appropriate after reaction to give the corresponding oxiranecarboxylic acid.

**Revendications**

**1.** Procédé pour préparer des alcools oxiranniques à haute pureté énantiomère, caractérisé en ce qu'on recristallise un mélange d'alcools oxiranniques isomères dans lequel l'énantiomère souhaité est présent en un excès d'au moins 60 % ee, dans un solvant ou dans un mélange de solvants à une température de 0,05 à 10°C inférieure au point de fusion de l'alcool oxirannique.

**2.** Procédé selon la revendication 1, caractérisé en ce qu'on utilise comme alcool oxirannique un composé de formule (I)

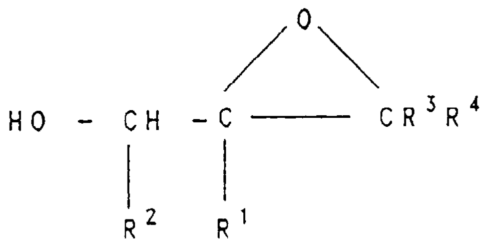

I

dans laquelle

R1 est un hydrogène ou un radical alkyle à chaîne droite ou ramifiée ayant de 1 à 18 atomes de carbone (avec ou sans atome de carbone asymétrique), où aussi un ou plusieurs groupes $-CH_2-$ peuvent être remplacés par des groupes -O-, -CO-, -CH=CH, -C≡C-, Δ, $-Si(CH_3)_2-$ ou 1,4-cyclohexylène, du moment que des atomes d'oxygène ne doivent pas être directement liés les uns aux autres, et du moment qu'au moins $R^3$ ou $R^4$ doit être différent d'un hydrogène quand $R^1$ n'est pas le radical méthyle, et du moment qu'aucun des radicaux -O-, -CO-, -CH=CH- ou -C≡C- ne peut être directement lié au noyau oxiranne, et/ou un ou plusieurs atomes d'hydrogène du radical alkyle peuvent être substitués par -F, -Cl, -Br, -SCN, -OCN ou $-N^3$,

$R^2$ est un hydrogène ou un radical alkyle à chaîne droite ou ramifiée ayant de 1 à 14 atomes de carbone (avec ou sans atome de carbone asymétrique), où aussi un ou plusieurs groupes $-CH_2-$ peuvent être remplacés par des groupes -O-, Δ, $-Si(CH_3)_2-$ ou 1,4-cyclohexylène, du moment que des atomes d'oxygène ne peuvent être directement liés les uns aux autres, et/ou un ou plusieurs atomes d'hydrogène du radical alkyle peuvent être substitués par -F, -Cl, -SCN, -OCN ou $-N_3$,

$R^3$ et $R^4$ sont des hydrogènes ou un radical alkyle à chaîne droite ou ramifiée ayant de 1 à 18 atomes de carbone (avec ou sans atome de carbone asymétrique), où aussi un ou plusieurs groupes $-CH_2-$ peuvent être remplacés par des groupes -O-, -CO-, -CH=CH, -C≡C-, Δ, $-Si(CH_3)_2-$ ou 1,4-cyclohexylène, du moment que des atomes d'oxygène ne doivent pas être directement liés les uns aux autres, et du moment que soit aucun des radicaux -O-, -CO-, -CH=CH- ou -C≡C- ne peut être directement lié au noyau oxiranne, et soit un ou plusieurs atomes d'hydrogène du radical alkyle peuvent être substitués par -F, -Cl, -Br, -SCN, -OCN ou $-N_3$,

$R^1$ et $R^3$, $R^1$ et $R^4$, ainsi que $R^3$ et $R^4$, peuvent aussi former ensemble un cycle.

**3.** Procédé selon la revendication 1 ou 2, caractérisé en ce qu'on utilise au moins un solvant choisi parmi l'ensemble comprenant les hydrocarbures aliphatiques et aromatiques, les hydrocarbures aliphatiques et aromatiques halogénés, les éthers, les esters et les amides.

**4.** Procédé pour préparer des dopants pour mélanges de cristaux liquides ferroélectriques, caractérisé en ce qu'on recristallise un mélange d'alcools oxiranniques isomères de formule (I) selon la revendication 2, dans lequel l'énantiomère souhaité est présent en un excès d'au moins 60% ee, dans un solvant ou un mélange de solvants à une température de 0,05 à 10°C inférieure au point de fusion de l'alcool oxirannique, puis, éventuellement après réaction conduisant à l'acide oxirannecarboxylique correspondant, on le fait réagir par éthérification ou estérification pour obtenir un dopant pour cristaux liquides ferroélectriques.